(19) Europäisches Patentamt
European Patent Office
Office européen des brevets

(11) Veröffentlichungsnummer: **0 177 830**
**A1**

(12) **EUROPÄISCHE PATENTANMELDUNG**

(21) Anmeldenummer: **85112062.6**

(51) Int. Cl.⁴: **A 01 N 47/38**, C 07 D 233/61

(22) Anmeldetag: **24.09.85**

(30) Priorität: **29.09.84 DE 3435944**

(43) Veröffentlichungstag der Anmeldung: **16.04.86**
**Patentblatt 86/16**

(84) Benannte Vertragsstaaten: **BE CH DE FR GB IT LI NL**

(71) Anmelder: **BASF Aktiengesellschaft,**
**Carl-Bosch-Strasse 38, D-6700 Ludwigshafen (DE)**

(72) Erfinder: **Rentzea, Costin, Dr.,**
**Richard-Kuhn-Strasse 1-3, D-6900 Heidelberg (DE)**
Erfinder: **Spiegler, Wolfgang, Dr.,**
**Westpreussenstrasse 5, D-6520 Worms 27 (DE)**
Erfinder: **Himmele, Walter, Dr., Eichenweg 14,**
**D-6909 Walldorf (DE)**
Erfinder: **Rademacher, Wilhelm, Dr., Austrasse 1,**
**D-6703 Limburgerhof (DE)**
Erfinder: **Jung, Johann, Prof. Dr., Hardenburgstrasse 19,**
**D-6703 Limburgerhof (DE)**

(54) **Vinyl-carbamoyl-imidazole enthaltende Mittel zur Regulierung des Pflanzenwachstums.**

(57) Mittel zur Regulierung des Pflanzenwachstums, enthaltend ein Vinyl-carbamoyl-imidazol der Formel

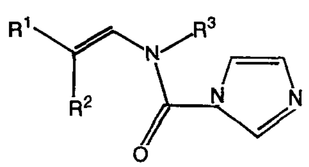

in der $R^1$ und $R^2$ gleich oder verschieden sind und Wasserstoff oder einen Alkylrest jeweils mit bis zu 6 Kohlenstoffatomen bedeuten
oder
$R^1$ zusammen mit $R^2$ einen 5- oder 6gliedrigen carbocyclischen Ring bildet
und
$R^3$ einen Alkyl- oder Cycloalkylrest jeweils mit bis zu 8 Kohlenstoffatomen bedeutet
und ein Verfahren zur Regulierung des Pflanzenwachstums unter Verwendung des Mittels.

EP 0 177 830 A1

Vinyl-carbamoyl-imidazole enthaltende Mittel zur Regulierung des Pflanzenwachstums

Die Erfindung betrifft Mittel zur Regulierung des Pflanzenwachstums, die ein Vinyl-carbamoyl-imidazole enthalten, sowie die Verwendung dieser Verbindungen zur Regulierung bzw. Beeinflussung des Pflanzenwachstums.

Es ist bekannt, daß bestimmte 2-Halogen-ethyl-trialkylammonium-halogenide pflanzenwachstumsregulierende Eigenschaften aufweisen (vgl. US-PS 3 156 554). So läßt sich mit Hilfe von (2-Chlorethyl)-trimethylammoniumchlorid das Pflanzenwachstum beeinflussen. Allerdings ist die Wirksamkeit dieses Stoffes, vor allem bei niedrigen Aufwandmengen, nicht immer ausreichend.

Es ist ferner bekannt, Triazol-Derivate wie das 3,3-Dimethyl-2-(1,2,4--triazolyl-(1))-1-(4-chlorbenzoyl)butan zur Regulierung des Pflanzenwachstums zu verwenden (DE-OS 27 39 352).

Es wurde nun gefunden, daß Vinyl-imidazolylharnstoffe der Formel I

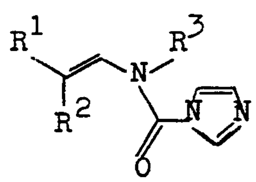

(I)

in der $R^1$ und $R^2$ gleich oder verschieden sind und Wasserstoff oder einen Alkylrest jeweils mit bis zu 6 Kohlenstoffatomen bedeuten oder $R^1$ und $R^2$ zusammen einen 5- oder 6-gliedrigen carbocyclischen Ring bilden und $R^3$ einen Alkyl- oder Cycloalkylrest jeweils mit bis zu 8 Kohlenstoffatomen bedeutet, hervorragend zur Beeinflussung des Pflanzenwachstums geeignet und sehr gut pflanzenverträglich sind.

Die Verbindungen enthalten Doppelbindungen und gegebenenfalls chirale Zentren je nach der Natur der Substituenten $R^1$, $R^2$ und/oder $R^3$ und werden im allgemeinen in Form von Z- und E-Isomerengemischen und gegebenenfalls als Racemate oder als Diastereomerengemische erhalten. Die Z- und E-Isomeren lassen sich bei einigen der erfindungsgemäßen Verbindungen beispielsweise durch Säulenchromatographie oder aufgrund von Löslichkeitsunterschieden in reiner Form isolieren. Aus einheitlichen Diastereomeren kann man ferner mit bekannten Methoden einheitliche Racemate und Enantiomere erhalten. Diese werden von der vorliegenden Erfindung eben-Mu/P

falls umfaßt. Für die Anwendung der erfindungsgemäßen Verbindungen sind sowohl die einheitlichen Diasteromeren, Enantiomeren bzw. geometrische Isomeren Z und E wie auch deren bei der Synthese anfallenden Gemische geeignet. Bevorzugt werden die letzteren verwendet.

$R^1$ und $R^2$ bedeuten beispielsweise Wasserstoff, Methyl, Ethyl, Propyl, Isopropyl, n-Butyl, sek.-Butyl, tert.-Butyl, n-Pentyl, 2-Methylbutyl, 2,2-Dimethylpropyl, n-Hexyl oder $R^1$ bildet zusammen mit $R^2$ einen Cyclopentyl- oder hexylring unter Einschluß des sie verbindenden C-Atoms.

Als Bedeutung für $R^3$ in Formel I kommen Reste wie n-Butyl, sek.-Butyl, iso-Butyl, tert.-Butyl, 2-Methylbutyl, 1,1,2-Trimethylpropyl-1, 3-Ethyl-pentyl-3, Cyclohexyl, 1-Methyl-1-cyclohexyl oder 1-Ethyl-1-cyclohexyl in Frage.

Man erhält die Vinyl-imidazolylharnstoffe der Formel 5 beispielsweise durch Umsetzung einem Vinyl-carbamoylchlorids der Formel II

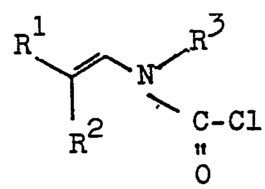

(II) ,

in der $R^1$, $R^2$ und $R^3$ die oben angegebenen Bedeutungen haben, a) mit Imidazol, b) mit Lithium, Natrium- oder Kalium-imidazolid oder c) mit 1-(Trimethylsilyl)-imidazol.

Die Umsetzungen a) und b) werden bevorzugt.

Herstellbeispiel 1

Zu einer Lösung von 18,1 g (0,26 Mol) Imidazol in 150 ml trockenem Tetrahydrofuran werden bei 20°C 20,3 g (0,1 Mol) N-(2-Isopropylvinyl)-N-tert.-butylcarbamoylchlorid zugetropft. Nach 8-stündigem Rühren bei 70°C wird das Gemisch auf 20°C abgekühlt und der gebildete Niederschlag abgesaugt. Das Filtrat wird unter vermindertem Druck eingeengt, der Rückstand in 200 ml Methylenchlorid aufgenommen, dreimal mit je 80 ml Wasser gewaschen, getrocknet und eingeengt. Das zurückbleibende Öl wird in Vakuum destilliert.

Man erhält 17,2 g 1-(n-Isopropylvinyl)-N-tert.-butylcarbamoyl)-imidazol als farblose Flüssigkeit vom Sdp. 105-106°C/0,1 mbar und $n_D^{25}$ 1,4893 (Verbindung Nr. 1).

In entsprechender Weise wurden die in der folgenden Tabelle physikalisch gekennzeichneten Verbindungen hergestellt; soweit keine physikalischen Merkmale angegeben sind, können diese Verbindungen aus entsprechenden Rohstoffen unter Verwendung der vorstehenden Versuchsangaben erhalten werden.

| Beisp. Nr. | $R^1$ | $R^2$ | $R^3$ | phys. Konstante |
|---|---|---|---|---|
| 2 | $CH_3$- | H | tert.-Butyl | Sdp. 90-91°C/0,2 mbar |
| 3 | $C_2H_5$- | H | tert.-Butyl | Sdp. 90-93°C/0,15 mbar |
| 4 | n-$C_3H_7$- | H | tert.-Butyl | Sdp. 115-117°C/0,3 mbar |
| 5 | $CH_3$- | $CH_3$- | tert.-Butyl | Sdp. 112-113°C/0,2 mbar |
| 6 | $C_2H_5$- | $C_2H_5$- | tert.-Butyl | $n_D^{25}$ 1,5030 |
| 7 | n-$C_4H_9$- | $C_2H_5$- | tert.-Butyl | $n_D^{25}$ 1,4990 |
| 8 | $-(CH_2)_5-$ | | tert.-Butyl | Fp. 73-76°C |
| 9 | i-$C_3H_7$- | H | n-Butyl | |
| 10 | i-$C_3H_7$- | H | n-Hexyl | |
| 11 | i-$C_3H_7$- | H | $-C(CH_3)_2CH_2CH_3$ | $n_D^{25}$ 1,4935 |
| 12 | i-$C_{37}$- | H | $-C(C_2H_5)_3$ | Fp. 54-56°C |
| 13 | i-$C_3H_7$- | H | $-C(CH_3)_2CH(CH_3)_2$ | $n_D^{25}$ 1,4970 |
| 14 | i-$C_3H_7$- | H | $-C(CH_3)_2CH_2CH_2CH_3$ | $n_D^{25}$ 1,4915 |
| 15 | I-$C_3H_7$- | H | 1-Ethyl-1-Cyclohexyl | $n_D^{25}$ 1,5105 |

Die Verbindungen der Formel I können praktisch alle Entwicklungsstadien einer Pflanze verschiedenartig beeinflussen und können deshalb ganz allgemein als Wachstumsregulatoren bezeichnet werden. Für die Wirkungsweise von Pflanzenwachstumsregulatoren gilt nach der bisherigen Erfahrung, daß ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation,

d) von den geoklimatischen Faktoren, z.B. Sonnenschein, Dauer, Durchschnittstemperatur, Niederschlagsmenge,

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumshemmern läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch

die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wirkstoffen der Formel I lassen sich Mehrerträge sowohl an Pflanzenteilen als auch an Pflanzeninhaltsstoffen erzielen. So es es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermgöglicht wird. Derselbe Mechanismus, das heißt zwischen Frucht- bzw. Blatt und Sproßteil der Pflanze ist auch für ein gut kontrollierbares Entblättern der Bäume wesentlich.

Die Wirkung der Verbindungen der Formel I ist besser als bei bekannten Wachstumsregulatoren. Die Wirkung zeigt sich sowohl bei Monokotylen, z.B. Getreide wie Weizen, Gerste, Roggen, Hafer und Reis oder Mais oder Gräsern als auch insbesondere bei Dikotylen (z.B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,1 bis 12 kg/ha bevorzugt 0,25 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten.

Anwendungsbeispiel A

Vorlaufbehandlung bei Sommergerste

Zur Bestimmung der wachstumsregulierenden Eigenschaft der Prüfsubstanzen wurden Testpflanzen auf ausreichend mit Nährstoffen versorgtem Torfkultursubstrat angezogen. Im Vorauflaufverfahren wurden die Wirksubstanzen in wäßriger Aufbereitung am Tage der Einsaat auf das Saatbett gegossen.

Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente Chlorcholinchlorid (CCC).

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind der.folgenden Tabelle zu entnehmen.

Sommergerste, Sorte "Aramir"
Vorauflaufverfahren

| Testsubstanz Beispiele | Konz. mg WS/Gef. | Wuchshöhen relativ |
|---|---|---|
| - | - | 100 |
| CCC | 6 | 80,1 |
| Verbindung | | |
| Nr. 1 | 6 | 70,6 |
| Nr. 2 | 6 | 78,5 |
| Nr. 3 | 6 | 78,5 |
| Nr. 4 | 6 | 78,5 |
| Nr. 5 | 6 | 78,5 |
| Nr. 13 | 6 | 73,6 |

## Anwendungsbeispiel B

Reiskeimlingstest

Junge Reiskeimlinge (Sorte Girona) wurden in einer Nährlösung kultiviert, die die jeweiligen Wirkstoffe in unterschiedlichen Konzentrationen enthielt. Nach sechs Tagen Kultur bei 25°C im Dauerlicht wurde die Wirkstoffkonzentration bestimmt, die das Längenwachstum der zweiten Blattscheide um 50 % vermindert (= $KI_{50}$). (Details bei: W. Rademacher und J. Jung, Berichte aus dem Fachgebiet Herbologie, Heft 24, pp 127-134, Universität Hohenheim, 1983)

| Testsubstanz | | $KI_{50}$ (molar) |
|---|---|---|
| CCC | | $1{,}5 \times 10^{-2}$ |
| Verbindung Nr. | 1 | $1{,}3 \times 10^{-5}$ |
| | 2 | $1{,}6 \times 10^{-4}$ |
| | 3 | $6{,}3 \times 10^{-5}$ |
| | 4 | $1{,}3 \times 10^{-5}$ |
| | 5 | $2{,}8 \times 10^{-4}$ |
| | 6 | $1{,}5 \times 10^{-5}$ |
| | 7 | $8{,}9 \times 10^{-5}$ |
| | 8 | $2{,}0 \times 10^{-5}$ |
| | 9 | |
| | 10 | |
| | 11 | $3{,}0 \times 10^{-6}$ |
| | 12 | $1{,}8 \times 10^{-5}$ |
| | 13 | $1{,}0 \times 10^{-5}$ |
| | 14 | $1{,}8 \times 10^{-6}$ |
| | 15 | $1{,}1 \times 10^{-5}$ |

## Anwendungsbeispiel C

Saatgutbehandlung bei Sommergerste

Saatgut von Gerste (Sorte Aramir) wurde mit den jeweiligen Wirkstoffen beladen. Die Aufwandmenge lag dabei - je nach Molekulargewicht - im Bereich von 0,5 Gramm pro Kilogramm Saatgut. (Der genaue Wert in g/kg ergibt sich durch Multiplikation des Molekulargewichts mit dem Faktor 2,35.) Die Kultivation der Pflanzen erfolgte in Torfkultursubstrat, dem ausreichend Nährstoffe zugesetzt waren, unter Gewächshausbedingungen. Nach 12 Tagen wurden die erreichten Sproßlängen in % der

unbehandelten Kontrollpflanzen erfaßt. Bei diesem Test erzielt z.B. die Verbindung Nr. 7 eine Verkürzung der Sproßlänge auf 5 %, während mit Chlorcholinchlorid praktisch kein Einfluß festzustellen ist.

Patentansprüche

1. Mittel zur Regulierung des Pflanzenwachstums, enthaltend ein Vinyl-carbamoyl-imidazol der Formel

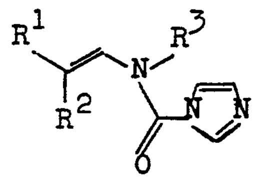

I,

in der $R^1$ und $R^2$ gleich oder verschieden sind und Wasserstoff oder einen Alkylrest jeweils mit bis zu 6 Kohlenstoffatomen bedeuten

oder

$R^1$ und $R^2$ zusammen einen 5- oder 6-gliedrigen carboyclischen Ring bildet

und

$R^3$ einen Alkyl- oder Cycloalkylrest jeweils mit bis zu 8 Kohlenstoffatomen bedeutet.

2. Mittel zur Regulierung des Pflanzenwachstums gemäß Anspruch 1 und 2, enthaltend einen flüssigen oder festen Trägerstoff und gegebenenfalls ein oder mehrere oberflächenaktive Mittel.

3. Verfahren zur Regulierung des Pflanzenwachstums, dadurch gekennzeichnet, daß man Mittel gemäß Anspruch 1 auf Pflanzen oder deren Lebensraum einwirken läßt.

4. Verfahren gemäß Anspruch 4, dadurch gekennzeichnet, daß man Mittel gemäß Anspruch 1 auf Pflanzensamen einwirken läßt.

5. Verwendung von Vinyl-carbamoyl-imidazolen der Formel I aus Anspruch 1 zur Regulierung des Pflanzenwachstums.

0177830

Europäisches Patentamt

# EUROPÄISCHER RECHERCHENBERICHT

Nummer der Anmeldung

EP 85 11 2062

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 139 365 (L.G. COPPING et al.)<br>* Zusammenfassung *<br>----- | 1-5 | A 01 N 47/38<br>C 07 D 233/61 |
| | | | RECHERCHIERTE SACHGEBIETE (Int. Cl.4) |
| | | | A 01 N<br>C 07 D |

Der vorliegende Recherchenbericht wurde für alle Patentansprüche erstellt.

| Recherchenort | Abschlußdatum der Recherche | Prüfer |
|---|---|---|
| DEN HAAG | 07-01-1986 | DECORTE D. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82